(19) Europäisches Patentamt
European Patent Office
Office européen des brevets

(11) Veröffentlichungsnummer: **0 458 327 B1**

(12) **EUROPÄISCHE PATENTSCHRIFT**

(45) Veröffentlichungstag der Patentschrift: **09.08.95**

(51) Int. Cl.[6]: **C07C 409/40**, C11D 3/39

(21) Anmeldenummer: **91108344.2**

(22) Anmeldetag: **23.05.91**

# (54) Ureidoperoxicarbonsäuren, Verfahren zu deren Herstellung und deren Verwendung.

(30) Priorität: **25.05.90 DE 4016980**

(43) Veröffentlichungstag der Anmeldung:
**27.11.91 Patentblatt 91/48**

(45) Bekanntmachung des Hinweises auf die Patenterteilung:
**09.08.95 Patentblatt 95/32**

(84) Benannte Vertragsstaaten:
**AT BE CH DE ES FR GB IT LI NL**

(56) Entgegenhaltungen:
**NL-A- 6 717 087**
**US-A- 4 634 551**

(73) Patentinhaber: **HOECHST AKTIENGESELLSCHAFT**

**D-65926 Frankfurt am Main (DE)**

(72) Erfinder: **Gethöffer, Hanspeter, Dr.**
**c/o Hoechst Italia S.p.A,**
**Strada Pianezza, 313**
**I-10151 Torino (IT)**
Erfinder: **Reinhardt, Gerd, Dr.**
**Freiherr-vom-Stein-Strasse 37**
**W-6233 Kelkheim (Taunus) (DE)**
Erfinder: **Naumann, Peter**
**Eichendorffstrasse 7**
**W-6204 Taunusstein 1 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Anorganische Persalze sind seit langem als Bleichzusätze in Waschmitteln bekannt. Da sie ihre optimale Bleichkraft jedoch erst bei Temperaturen oberhalb 60 °C entfalten, werden zu ihrer Aktivierung eine Reihe von organischen Verbindungen beschrieben, die während des Waschprozesses mit Wasserstoffperoxid unter Freisetzung einer Peroxicarbonsäure reagieren, welche bereits bei 40-60 °C bleichend wirkt. Eine Übersicht zahlreicher bekannter Perborataktivatoren wie N-Acylverbindungen (Tetraacetylethylendiamin, Tetraacetylmethylendiamin, Tetraacetylglycoluril) oder aktivierte Ester (Pentaacetylglucose, Acetoxibenzolsulfonat-Natrium, Benzoyloxibenzolsulfonat-Natrium) ist z.B. in US 4,248,928 gegeben.

Daneben werden in neuerer Zeit eine Reihe organischer Peroxicarbonsäuren als Bleichsysteme für Waschmittel beschrieben. Neben bereits kommerziell erhältlichen Peroxicarbonsäuren wie Dodecandiperoxicarbonsäure (EP 127 782) und Monoperoxiphthalsäure (EP 27 693) sind Perbernsteinsäure (DE 34 38 529), Perglutarsäure (DE 35 39 036), und Sulfoperbenzoesäure beschrieben. Problematisch an diesen Peroxicarbonsäuren ist jedoch ihre geringe Lagerstabilität, die zum Teil erst durch besondere physikalische oder chemische Stabilisierung gewährleistet wird. Insbesondere hat sich hier die Herstellung von Magnesium-Salzen (EP 105 689) oder ein Zusatz von Phosphanoxid/Natriumsulfat (DE 33 20 497) bewährt. Nach EP 170 386 und US-4,634 551 können organische Peroxicarbonsäuren auch durch eine zusätzliche Amidgruppe, nach EP 349 940 auch durch eine zusätzliche Imidgruppe im Molekül stabilisiert werden. Darüberhinaus werden zahlreiche weitere Peroxicarbonsäuren, die stabilisierende funktionelle Gruppen besitzen, beschrieben, so z.B. Ammoniumperoxicarbonsäuren (EP 316 809) Pyridin-N-oxidperoxicarbonsäuren (EP 300 461) oder Sulfonperoxicarbonsäuren (EP 267 175).

Gegenstand der vorliegenden Erfindung sind Peroxicarbonsäuren der allgemeinen Formel

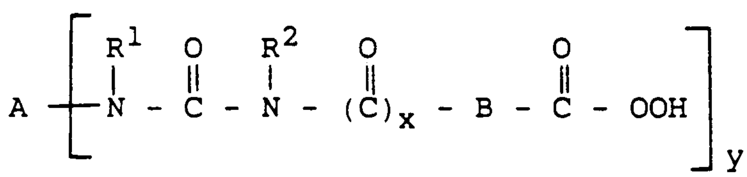

worin

y — die Zahlen 1 oder 2

A — falls y = 1 ist
Wasserstoff, $C_1$-$C_2$-Alkyl, Aryl, bevorzugt Phenyl oder eine Gruppe der Formel

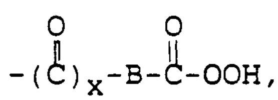

falls y = 2 ist,
$C_2$-$C_{20}$-Alkylen oder Arylen, bevorzugt Phenylen bedeuten,

$R^1$ und $R^2$ — gleich oder verschieden sein können und Wasserstoff, $C_1$-$C_{20}$-Alkyl, Aryl, bevorzugt Phenyl oder Halogenaryl, bevorzugt Chlorphenyl, oder Alkoxyaryl, bevorzugt $C_1$-$C_4$-Alkoxyphenyl, oder Alkylaryl, bevorzugt $C_1$-$C_4$-Alkylphenyl, bedeuten
oder $R^1$ und $R^2$ auch gemeinsam einen $C_2$-$C_4$-Alkylenrestdarstellen können,

x — die Zahlen 0 oder 1,

B — eine Gruppe der Formeln
$-(CH_2)_n-$,

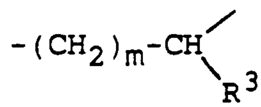,
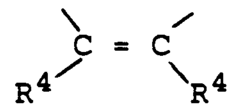

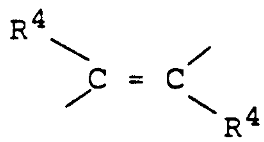
oder
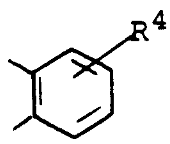

| | |
|---|---|
| n | die Zahlen von 1 bis 20, |
| m | die Zahlen 0, 1 oder 2, |
| $R^3$ | $C_1$-$C_{20}$-Alkyl und |
| $R^4$ | jeweils Wasserstoff oder $C_1$-$C_{20}$-Alkyl |

bedeuten.

Insbesondere bevorzugt sind N-Carbamoyl-6-aminoperoxihexansäure, N-Carbamoyl-11-aminoperoxiundekansäure, N-(N'-Phenylcarbamoyl)-6-aminoperoxihexansäure, N-Carbamoylperoxibernsteinsäuremonoamid, N-(N'-Butylcarbamoyl)peroxibernsteinsäuremono-amid, N-Carbamoylperoxiglutarsäuremonoamid und N-(N'-Butylcarbamoyl)-peroxiglutarsäuremonoamid.

Die Herstellung der Ureidoperoxicarbonsäuren erfolgt durch die Schritte:

-a- Synthese der Ureidocarbonsäuren,
-b- Oxidation zur Ureidoperoxicarbonsäure,
-c- Isolierung der Ureidoperoxicarbonsäure

Im folgenden werden die einzelnen Schritte näher erläutert. Die Herstellung der Ureidocarbonsäuren

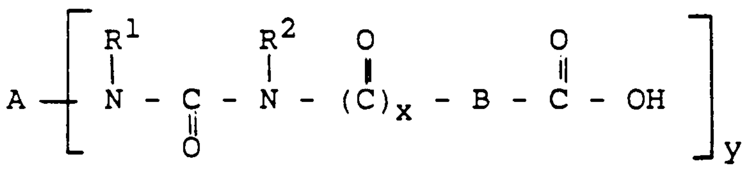

kann durch an sich bekannte Syntheseschritte erfolgen. Die Verbindungen mit y = 1 und x = 0 werden durch Umsetzen von Kaliumisocyanat oder Isocyanaten der Formel

$R^1 - N = C = O$

mit Aminocarbonsäuren der Formel

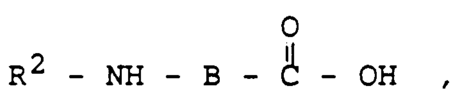,

Verbindungen mit y = 2 und x = 0 durch Reaktion von Diisocyanaten der Formel

$O = C = N - A - N = C = O$

mit zwei Äquivalenten Aminocarbonsäure erhalten (s. Houben-Weyl, Methoden der Organischen Chemie, VIII, S. 157). Ureidocarbonsäuren mit y = 1 und x = 1 sind durch Synthesen ausgehend von Anhydriden der Formel

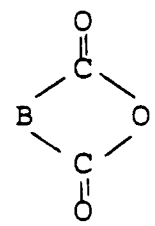

und Harnstoff, bzw. substituierten Harnstoffen der Formel

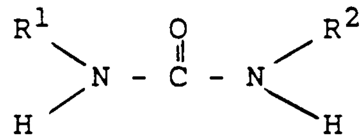

,

Ureidocarbonsäuren mit y = 2 und x = 1 durch Verwendung von zwei Äquivalenten Anhydrid pro Äquivalent Harnstoff bzw. subst. Harnstoff zugänglich (s. U.S. 2 717 908, DP 945 234).

Als Isocyanate können insbesondere Phenylisocyanat, p-Tolylisocyanat und Hexamethylendiisocyanat als Aminocarbonsäuren Glycin, $\alpha$- und $\beta$-Alanin, $\epsilon$-Aminocapronsäure und $\omega$-Aminoundekancarbonsäure, als Anhydride Bernsteinsäureanhydrid, Glutarsäureanhydrid, Maleinsäureanhydrid, Phthalsäureanhydrid oder Alkylbernsteinsäureanhydrid und als Harnstoffverbindungen Harnstoff, N-Butylharnstoff, N-Octylharnstoff und N-Phenylharnstoff eingesetzt werden.

Die Umwandlung der im Schritt -a- erhaltenen Ureidocarbonsäuren zu Ureidoperoxicarbonsäuren erfolgt durch Umsetzung mit einem Oxidationsgemisch aus Wasserstoffperoxid und einer starken Säure. Wasserstoffperoxid kommt als 30 bis 95 gew.-prozentige, vorzugsweise 50 bis 85 gew.-prozentige, wäßrige Lösung zum Einsatz. Geeignete saure Katalysatoren sind Schwefelsäure, Methansulfonsäure oder ein saurer Ionenaustauscher. Schwefelsäure wird als 50 bis 96 gew.-prozentige, vorzugsweise 75 bis 96 gew.-prozentige wäßrige Lösung verwendet.

Wasserstoffperoxid wird pro oxidierbarer Carboxylgruppe der Ureidocarbonsäure im molaren Verhältnis 10 bis 1 zu 1, vorzugsweise 4 bis 2 zu 1 eingesetzt. Art und Menge der Katalysatorsäure sind abhängig von der eingesetzten Ureidocarbonsäure. Im allgemeinen wird die 2 bis 6-fache Gewichtsmenge - bezogen auf die Ureidocarbonsäure - an Katalysatorsäure zugesetzt. Die Reaktionstemperatur richtet sich nach der Stabilität der entsprechenden Ureidoperoxicarbonsäure und liegt zwischen 5 und 30 ° C, vorzugsweise 10 bis 20 ° C.

Die beanspruchten Ureidoperoxicarbonsäuren fallen im allgemeinen durch Zugabe von Wasser aus und können in einfacher Weise durch Filtration oder Zentrifugation isoliert werden. Es ist auch möglich, Ureidoperoxicarbonsäuren, die durch Wasserzugabe nicht oder nur unvollständig ausfallen, durch Zugabe von wäßrigen Lösungen von basischen Salzen auszufällen.

Die erfindungsgemäßen Ureidoperoxicarbonsäuren sind fest, nahezu geruchlos, besitzen einen niedrigen Dampfdruck und sind von ausgezeichneter thermischer Stabilität. Sie können als Lösungen , Pulver oder in verarbeiteter Form allein oder in Kombination mit weiteren Stoffen zu Bleich-, Oxidations- oder Desinfektionszwecken eingesetzt werden.

**Beispiele**

**Beispiel 1** N-Carbamoyl-11-aminoperoxiundekansäure

61.6 g (0,25 Mol) N-Carbamoyl-11-aminoundekansäure werden in 74.4 g Schwefelsäure (96 gew.-%ig) gelöst und auf 15 ° C gekühlt. Unter Kühlen werden 23.8 g (0,6 Mol) Wasserstoffperoxid (85 gew.-%ig) so zugetropft, daß die Innentemperatur zwischen 15 und 20 ° C gehalten werden kann. Die Reaktionsmischung wird unter gelegentlichem Kühlen noch 1 h bei 20 ° C Innentemperatur gerührt, auf 10 ° C abgekühlt, mit 200 ml Wasser tropfenweise versetzt und die ausgefallene Peroxicarbonsäure abgesaugt. Der Filterkuchen wird mit Wasser mineralsäurefrei gewaschen und bei 40 ° C im Wasserstrahlvakuum getrocknet.

Ausbeute: 63.3 g (96.5 %), weiße geruchlose Kristalle

Aktivsauerstoffgehalt: 5.8 % (94,6 %)

Fp.: 99-101 ° C

**Beispiel 2** N-Carbamoyl-6-aminoperoxihexansäure

17.4 g (0.1 Mol) N-Carbamoyl-6-aminohexansäure werden in 50 g Methansulfonsäure gelöst und auf 15 ° C gekühlt. Unter Kühlen werden 12 g (0.3 Mol) Wasserstoffperoxid (85 gew.-%ig) so zugetropft, daß die Innentemperatur zwischen 15 und 20 ° C gehalten werden kann. Es wird noch 10 min bei 18 ° C gerührt, auf 0 ° C abgekühlt und mit wäßriger Natriumhydroxidlösung (33 gew.-%ig) auf pH 3 gestellt. Es werden 65 g Natriumhydroxidlösung eingesetzt. Nach 20 minütigem Rühren bei 0 ° C wird die ausgefallene Peroxicarbonsäure abgenutscht, mit Eiswasser gewaschen und bei 40 ° C im Wasserstrahlvakuum getrocknet.
Ausbeute: 16.2 g (85 %)
Aktivsauerstoffgehalt: 5.0 % (60 %)
Zur Reinigung wird eine Probe zweimal aus Essigsäureethylester umkristallisiert (15 g umzukristallisierende Peroxicarbonsäure auf 1.5 l Essigsäureethylester)
Aktivsauerstoffgehalt: 7.2 % ( 86 %), weiße geruchlose Kristalle
Fp.: 160 ° C

**Beispiel 3** N-(Carbamoyl)peroxibernsteinsäuremonoamid

80 g (0.5 Mol) N-(Carbamoyl)bernsteinsäuremonoamid werden in 160 g Methansulfonsäure gelöst und auf 15 ° C gekühlt. Unter Kühlen werden 60 g (1.5 Mol) Wasserstoffperoxid (85 gew.-%ig) so zugetropft, daß die Innentemperatur zwischen 15 und 20 ° C gehalten werden kann. Die Reaktionsmischung wird 10 min bei 20 ° C gerührt, auf 10 ° C abgekühlt und tropfenweise mit Wasser unter Kühlung versetzt. Die ausgefallene Peroxicarbonsäure wird abgesaugt, der Nutschkuchen mit Wasser mineralsäurefrei gewaschen und bei 40 ° C im Wasserstrahlvakuum getrocknet.
Ausbeute: 53.1 g (60.3 %), weiße Kristalle
Aktivsauerstoffgehalt: 9 % (99 %)
Fp.: 176-179 ° C

**Beispiel 4** N-(Carbamoyl)peroxiglutarsäuremonoamid

11.3 g (0.065 Mol) N-(Carbamoyl)glutarsäuremonoamid, 21 g Methansulfonsäure und 7.8 g (0.2 Mol) Wasserstoffperoxid 85 gew.-%ig werden wie in Beispiel 3 beschrieben zur Reaktion gebracht und aufgearbeitet.
Ausbeute: 8.8 g (71.3 %), weiße Kristalle
Aktivsauerstoffgehalt: 8.2 % (98 %)
Fp.: 130 ° C

**Beispiel 5** N-(Carbamoyl)peroxidodecylbernsteinsäuremonoamid

6.1 g (0.019 Mol) N-(Carbamoyl)dodecylbernsteinsäuremonoamid, 30 g Methansulfonsäure und 2.3 g (0.056 Mol) Wasserstoffperoxid 85 gew.-%ig werden wie in Beispiel 3 beschrieben zur Reaktion gebracht und aufgearbeitet.
Ausbeute: 6.2 g (99 %), weiße Kristalle
Aktivsauerstoffgehalt: 4.7 % (100 %)
Fp.: 149 ° C

**Beispiel 6** N-(N'-Octylcarbamoyl)peroxibernsteinsäuremonoamid

13.6 g (0.05 Mol) N-(N'-Octylcarbamoyl)bernsteinsäuremonoamid, 50 g Methansulfonsäure und 6 g (0.15 Mol) Wasserstoffperoxid 85 gew.-%ig werden wie in Beispiel 3 beschrieben zur Reaktion gebracht und aufgearbeitet.
Ausbeute: 12.5 g (87 %), weiße Kristalle
Aktivsauerstoffgehalt: 5.5 % (98.3 %)
Fp.: 113 ° C

**Beispiel 7** N-(N'-Phenylcarbamoyl)-2-aminoperoxipropansäure

20.8 g (0.01 Mol) N-(N'-Phenylcarbamoyl)-2-aminopropansäure, 35 g Methansulfonsäure und 12 g (0.3 Mol) Wasserstoffperoxid 85 gew.-%ig werden wie in Beispiel 3 beschrieben zur Reaktion gebracht und aufgearbeitet.
Ausbeute: 19.5 g (87 %), leicht gelbe Kristalle
Aktivsauerstoffgehalt: 6.6 % (93 %)
Fp.: 147 °C

**Beispiel 8** N-(N'-Phenylcarbamoyl)-6-aminoperoxihexansäure

12.5 g (0.05 Mol) N-(N'-Phenylcarbamoyl)-6-aminohexansäure, 25 g Methansulfonsäure und 6 g (0.15 Mol) Wasserstoffperoxid (85 gew.-%ig) werden wie in Beispiel 3 beschrieben zur Reaktion gebracht und aufgearbeitet.
Ausbeute: 13.5 g (100 %), weiße Kristalle
Aktivsauerstoffgehalt: 5.6 % (93 %)
Fp.: 102 °C

**Beispiel 9** N-(N'-Butylcarbamoyl)-peroxiglutarsäuremonoamid

80.8 g (0.35 Mol) N-(N'-Butylcarbamoyl)glutarsäuremonoamid, 123 g Methansulfonsäure und 42 g (1.05 Mol) Wasserstoffperoxid 85 gew.-%ig werden wie in Beispiel 3 beschrieben zur Reaktion gebracht und aufgearbeitet.
Ausbeute: 34.6 g (40 %), weiße Kristalle
Aktivsauerstoffgehalt: 5.4 % (83 %)
Fp.: 173-75 °C

**Beispiel 10** N-(Carbamoyl)peroxiglutarsäuremonoamid

87 g (0.5 Mol) N-(Carbamoyl)glutarsäuremonoamid, 175 g Schwefelsäure und 102 g (1.5 Mol) Wasserstoffperoxid (50 gew.-%ig) werden wie in Beispiel 3 beschrieben zur Reaktion gebracht und aufgearbeitet.
Ausbeute: 64.5 g (75.8 %), weiße Kristalle
Aktivsauerstoffgehalt: 8.1 % (96.1 %)
Fp.: 133 °C

### 1. Waschversuche im Launder-o-meter

Die Waschversuche wurden im Launder-o-meter bei Temperaturen von 20, 40 und 60 °C unter Verwendung von Wasser der Wasserhärte 15 °dH durchgeführt. Die Waschzeit betrug 30 min. Als Waschmittel wurden 1.5 g/L phosphathaltiges IEC-Waschmittel zugesetzt. Der Persäuren wurden so dosiert, daß sie, bei vollständiger Auflösung, jeweils 25 mg Aktivsauerstoff (OA) freisetzen konnten. Als Standard-Anschmutzung dienten Tee auf Baumwolle (WFK) oder Rotwein auf Baumwolle (Empa). Die Bleichleistung ist als Remissionswert nach der Wäsche angegeben.

Bleichergebnisse an Tee-Anschmutzung

| | Waschtemperatur | | |
|---|---|---|---|
| Bleichsystem | 20°C | 40°C | 60°C |
| Verbindung nach Beispiel 2 | 58.4 | 67.7 | 71.4 |
| Verbindung nach Beispiel 6 | 57.4 | 70.6 | 75.6 |
| Verbindung nach Beispiel 9 | 60.8 | 69.2 | 73.8 |
| Verbindung nach Beispiel 10 | 59.7 | 68.7 | 72.0 |
| TAED/Perborat | | 65.7 | 67.9 |
| Perborat | | 58.6 | 62.8 |

Bleichergebnisse an Rotwein-Anschmutzungen

| | Waschtemperatur | | |
|---|---|---|---|
| Bleichsystem | 20°C | 40°C | 60°C |
| Verbindung nach Beispiel 2 | 63.0 | 70.6 | 75.8 |
| Verbindung nach Beispiel 6 | 62.1 | 71.0 | 79.4 |
| Verbindung nach Beispiel 9 | 64.5 | 72.1 | 77.7 |
| Verbindung nach Beispiel 10 | 64.9 | 71.6 | 76.6 |
| TAED/Perborat | | 65.5 | 67.9 |
| Perborat | | 61.2 | 64.6 |

**Patentansprüche**
**Patentansprüche für folgende Vertragsstaaten : AT, BE, CH, DE, FR, GB, IT, LI, NL**

**1.** Verbindungen der Formel

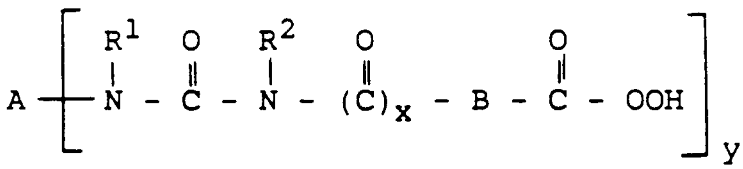

worin

| | |
|---|---|
| y | die Zahlen 1 oder 2 |
| A | falls y = 1 ist |
| | Wasserstoff, $C_1$-$C_2$-Alkyl, Aryl, oder eine Gruppe der Formel |

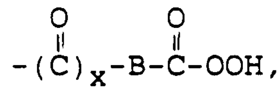

falls y = 2 ist,
$C_2$-$C_{20}$-Alkylen oder Arylen, bedeuten,

$R^1$ und $R^2$ gleich oder verschieden sein können und Wasserstoff, $C_1$-$C_{20}$-Alkyl, Aryl, Halogenaryl, Alkoxyaryl oder Alkylaryl, bedeuten
oder $R^1$ und $R^2$ auch gemeinsam einen $C_2$-$C_4$-Alkylenrestdarstellen können,

x die Zahlen 0 oder 1,

B eine Gruppe der Formeln
$-(CH_2)_n-$,

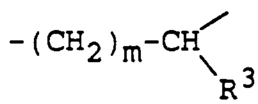 , 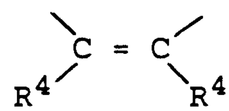

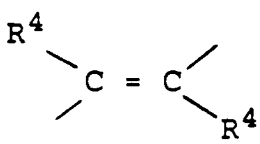 oder 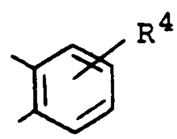

n die Zahlen von 1 bis 20,

m die Zahlen 0, 1 oder 2,

$R^3$ $C_1$-$C_{20}$-Alkyl und

$R^4$ jeweils Wasserstoff oder $C_1$-$C_{20}$-Alkyl

bedeuten.

**2.** Verbindungen nach Anspruch 1, worin y die Zahlen 1 oder 2

A falls y = 1 ist Wasserstoff, $C_1$-$C_2$-Alkyl, Phenyl oder eine Gruppe der Formel

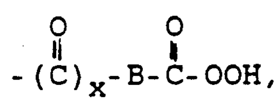

falls y = 2 ist,
$C_2$-$C_{20}$-Alkylen oder Phenylen bedeuten,

$R^1$ und $R^2$ gleich oder verschieden sein können und Wasserstoff, $C_1$-$C_{20}$-Alkyl, Phenyl, Chlorphenyl, $C_1$-$C_4$-Alkoxyphenyl oder $C_1$-$C_4$-Alkylphenyl, bedeuten oder $R^1$ und $R^2$ auch gemeinsam einen $C_2$-$C_4$-Alkylenrestdarstellen können,

x die Zahlen 0 oder 1,

B eine Gruppe der Formeln
$-(CH_2)_n-$,

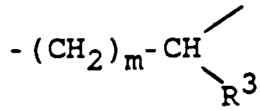 , 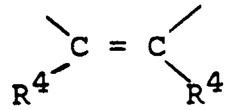

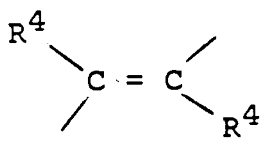 oder 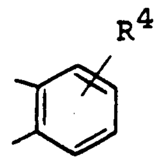

| | |
|---|---|
| n | die Zahlen von 1 bis 20, |
| m | die Zahlen 0, 1 oder 2, |
| $R^3$ | $C_1$-$C_{20}$-Alkyl und |
| $R^4$ | jeweils Wasserstoff oder $C_1$-$C_{20}$-Alkyl |

bedeuten.

**3.** Verbindungen nach Anspruch 1, worin y die Zahl 1, A Wasserstoff, Phenyl oder $C_1$-$C_2$-Alkyl, $R^1$ und $R^2$ Wasserstoff, x die Zahlen Null oder 1, B eine Gruppe der Formel $-(CH_2)_n-$ und n Zahlen von 1 bis 20 bedeuten.

**4.** Verbindungen nach Anspruch 1, worin y die Zahl 1, A Wasserstoff, Phenyl oder $C_1$-$C_2$-Alkyl, $R^1$ und $R^2$ Wasserstoff, x die Zahl Null, B eine Gruppe der Formel $-(CH_2)_n-$ und n die Zahlen von 1 bis 10, bevorzugt von 1 bis 6, bedeuten.

**5.** Verfahren zur Herstellung der Verbindungen nach mindestens einem der Ansprüche 1 bis 4, dadurch gekennzeichnet, daß eine Ureidocarbonsäure der Formel

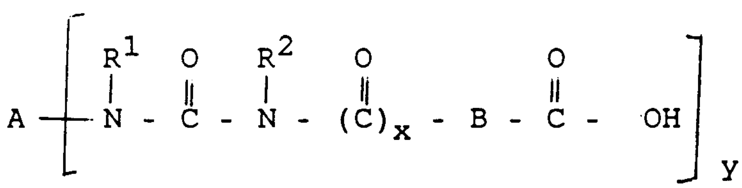

worin

| | |
|---|---|
| y | die Zahlen 1 oder 2 |
| A | falls y = 1 ist<br>Wasserstoff, $C_1$-$C_2$-Alkyl, Aryl, oder eine Gruppe der Formel |

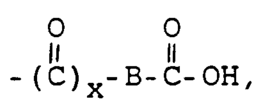

| | |
|---|---|
| | falls y = 2 ist,<br>$C_2$-$C_{20}$-Alkylen oder Arylen, bedeuten, |
| $R^1$ und $R^2$ | gleich oder verschieden sein können und Wasserstoff, $C_1$-$C_{20}$-Alkyl, Aryl, Halogenaryl, Alkoxyaryl, oder Alkylaryl, bedeuten<br>oder $R^1$ und $R^2$ auch gemeinsam einen $C_2$-$C_4$-Alkylenrestdarstellen können, |
| x | die Zahlen 0 oder 1, |
| B | eine Gruppe der Formeln<br>$-(CH_2)_n-$, |

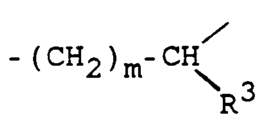 , 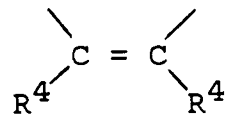

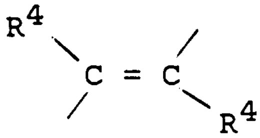 oder 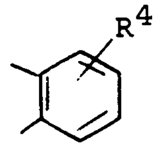

| | |
|---|---|
| n | die Zahlen von 1 bis 20, |
| m | die Zahlen 0, 1 oder 2, |
| $R^3$ | $C_1$-$C_{20}$-Alkyl und |
| $R^4$ | jeweils Wasserstoff oder $C_1$-$C_{20}$-Alkyl |

bedeuten, mit einem Oxidationsgemisch aus Wasserstoffperoxid mit einer starken Säure, vorzugsweise Schwefelsäure, Methansulfonsäure oder ein saurer Ionenaustauscher, umgesetzt wird, wobei Wasserstoffperoxid im molaren Verhältnis 10 bis 1 zu 1, vorzugsweise 4 bis 2 zu 1, pro oxidierbarer Carboxylgruppe der Ureidocarbonsäure und die 2 bis 6 fache Gewichtsmenge der starken Säure, bezogen auf die Ureidocarbonsäure, eingesetzt wird.

**6.** Verfahren nach Anspruch 5, dadurch gekennzeichnet, daß eine 30 bis 95 gew.-prozentige, vorzugsweise 50 bis 85 gew.-prozentige, wäßrige Wasserstoffperoxidlösung verwendet wird.

**7.** Verfahren gemäß Anspruch 5 oder 6, dadurch gekennzeichnet, daß als starke Säure 50 bis 96 gew.-prozentige, vorzugsweise 75 bis 96 gew.-prozentige, Schwefelsäure verwendet wird.

**8.** Verfahren gemäß einem der Ansprüche 5 bis 7, dadurch gekennzeichnet, daß die Reaktionstemperatur zwischen 5 und 30 ° C insbesondere zwischen 10 und 20 ° C, liegt.

**9.** Verwendung der Verbindungen nach Anspruch 1 als Bleich-, Oxidations- oder Desinfektionsmittel.

**Patentansprüche für folgenden Vertragsstaat: ES**

**1.** Bleich-, Oxidations- oder Desinfektionsmittel enthaltend Verbindungen der Formel

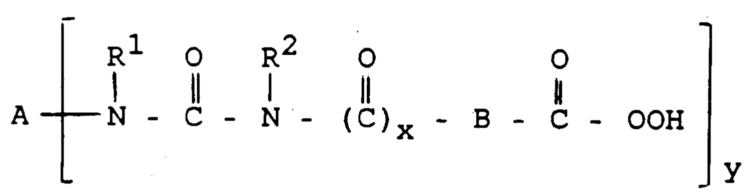

worin

| | |
|---|---|
| y | die Zahlen 1 oder 2 |
| A | falls y = 1 ist<br>Wasserstoff, $C_1$-$C_2$-Alkyl, Aryl, oder eine Gruppe der Formel |

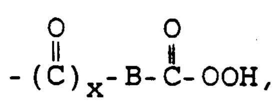

falls y = 2 ist,
$C_2$-$C_{20}$-Alkylen oder Arylen, bedeuten,

$R^1$ und $R^2$ gleich oder verschieden sein können und Wasserstoff, $C_1$-$C_{20}$-Alkyl, Aryl, Halogenaryl,

Alkoxyaryl oder Alkylaryl, bedeuten
oder $R^1$ und $R^2$ auch gemeinsam einen $C_2$-$C_4$-Alkylenrestdarstellen können,

x die Zahlen 0 oder 1,

B eine Gruppe der Formeln
$-(CH_2)_n-$,

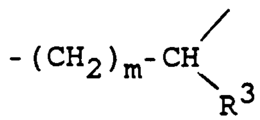 , 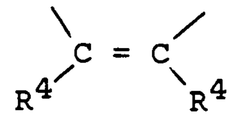

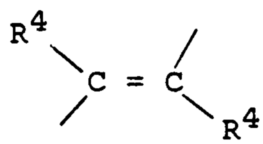 oder 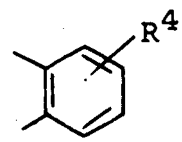

n die Zahlen von 1 bis 20,

m die Zahlen 0, 1 oder 2,

$R^3$ $C_1$-$C_{20}$-Alkyl und

$R^4$ jeweils Wasserstoff oder $C_1$-$C_{20}$-Alkyl

bedeuten.

**2.** Bleich-, Oxidations- oder Desinfektionsmittel enthaltend Verbindungen nach Anspruch 1, worin y die Zahlen 1 oder 2

A falls y = 1 ist Wasserstoff, $C_1$-$C_2$-Alkyl, Phenyl oder eine Gruppe der Formel

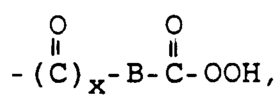

falls y = 2 ist,
$C_2$-$C_{20}$-Alkylen oder Phenylen bedeuten,

$R^1$ und $R^2$ gleich oder verschieden sein können und Wasserstoff, $C_1$-$C_{20}$-Alkyl, Phenyl, Chlorphenyl, $C_1$-$C_4$-Alkoxyphenyl oder $C_1$-$C_4$-Alkylphenyl, bedeuten
oder $R^1$ und $R^2$ auch gemeinsam einen $C_2$-$C_4$-Alkylenrestdarstellen können,

x die Zahlen 0 oder 1,

B eine Gruppe der Formeln
$-(CH_2)_n-$,

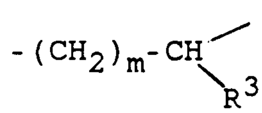 , 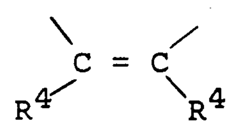

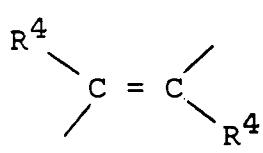 oder 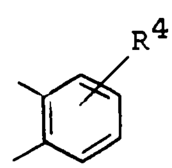

n die Zahlen von 1 bis 20,

m die Zahlen 0, 1 oder 2,

$R^3$ $C_1$-$C_{20}$-Alkyl und

$R^4$ jeweils Wasserstoff oder $C_1$-$C_{20}$-Alkyl

bedeuten.

3. Bleich-, Oxidations- oder Desinfektionsmittel enthaltend Verbindungen nach Anspruch 1, worin y die Zahl 1, A Wasserstoff, Phenyl oder $C_1$-$C_2$-Alkyl, $R^1$ und $R^2$ Wasserstoff, x die Zahlen Null oder 1, B eine Gruppe der Formel $-(CH_2)_n-$ und n Zahlen von 1 bis 20 bedeuten.

4. Bleich-, Oxidations- oder Desinfektionsmittel enthaltend Verbindungen nach Anspruch 1, worin y die Zahl 1, A Wasserstoff, Phenyl oder $C_1$-$C_2$-Alkyl, $R^1$ und $R^2$ Wasserstoff, x die Zahl Null, B eine Gruppe der Formel $-(CH_2)_n-$ und n die Zahlen von 1 bis 10, bevorzugt von 1 bis 6, bedeuten.

5. Verfahren zur Herstellung von Bleich-, Oxidations- oder Desinfektionsmittel enthaltend Verbindungen nach mindestens einem der Ansprüche 1 bis 4, dadurch gekennzeichnet, daß eine Ureidocarbonsäure der Formel

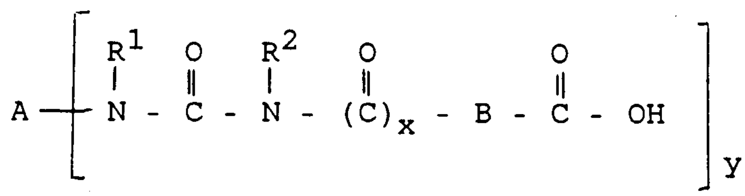

worin

y die Zahlen 1 oder 2

A falls y = 1 ist

Wasserstoff, $C_1$-$C_2$-Alkyl, Aryl, oder eine Gruppe der Formel

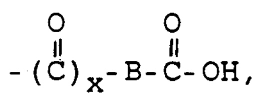

falls y = 2 ist,

$C_2$-$C_{20}$-Alkylen oder Arylen, bedeuten,

$R^1$ und $R^2$ gleich oder verschieden sein können und Wasserstoff, $C_1$-$C_{20}$-Alkyl, Aryl, Halogenaryl, Alkoxyaryl, oder Alkylaryl, bedeuten oder $R^1$ und $R^2$ auch gemeinsam einen $C_2$-$C_4$-Alkylenrest darstellen können,

x die Zahlen 0 oder 1,

B eine Gruppe der Formeln

$-(CH_2)_n-$,

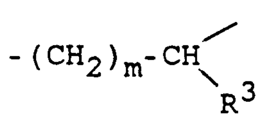 ,

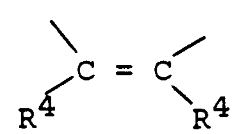

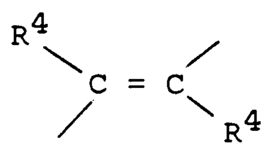 oder 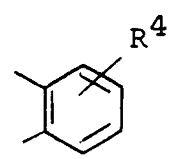

n die Zahlen von 1 bis 20,

m die Zahlen 0, 1 oder 2,

$R^3$ $C_1$-$C_{20}$-Alkyl und
$R^4$ jeweils Wasserstoff oder $C_1$-$C_{20}$-Alkyl
bedeuten,
mit einem Oxidationsgemisch aus Wasserstoffperoxid und einer starken Säure, vorzugsweise Schwefelsäure, Methansulfonsäure oder ein saurer Ionenaustauscher, umgesetzt wird, wobei Wasserstoffperoxid im molaren Verhältnis 10 bis 1 zu 1, vorzugsweise 4 bis 2 zu 1, pro oxidierbarer Carboxylgruppe der Ureidocarbonsäure und die 2 bis 6-fache Gewichtsmenge der starken Säure, bezogen auf die Ureidocarbonsäure, eingesetzt wird.

6. Verfahren zur Herstellung von Bleich-, Oxidations- oder Desinfektionsmittel enthaltend Verbindungen nach Anspruch 5, dadurch gekennzeichnet, daß eine 30 bis 95 gew.-prozentige, vorzugsweise 50 bis 85 gew.-prozentige, wäßrige Wasserstoffperoxidlösung verwendet wird.

7. Verfahren zur Herstellung von Bleich-, Oxidations- oder Desinfektionsmittel enthaltend Verbindungen gemäß Anspruch 5 oder 6, dadurch gekennzeichnet, daß als starke Säure 50 bis 96 gew.-prozentige, vorzugsweise 75 bis 96 gew.-prozentige Schwefelsäure verwendet wird.

8. Verfahren zur Herstellung von Bleich-, Oxidations- oder Desinfektionsmittel enthaltend Verbindungen gemäß einem der Ansprüche 5 bis 7, dadurch gekennzeichnet, daß die Reaktionstemperatur zwischen 5 und 30 °C, insbesondere zwischen 10 bis 20 °C, liegt.

**Claims**
**Claims for the following Contracting States: AT, BE, CH, DE, FR, GB, IT, LI, NL**

1. Compounds of the formula

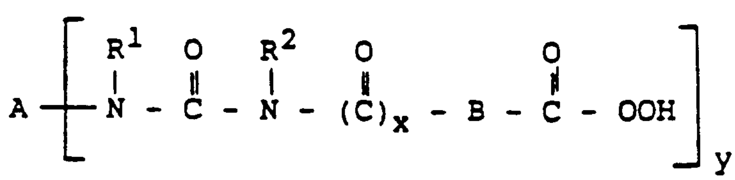

wherein
y is 1 or 2,
A is hydrogen, $C_1$-$C_2$-alkyl, aryl or a group of the formula

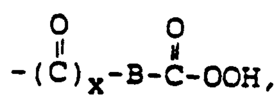

if y is 1, or $C_2$-$C_{20}$-alkylene or arylene if y is 2,
$R^1$ and $R^2$ can be identical or different and are each hydrogen, $C_1$-$C_{20}$-alkyl, aryl, haloaryl, alkoxyaryl or alkylaryl, or else $R^1$ and $R^2$ are together a $C_2$-$C_4$-alkylene radical,
x is 0 or 1,
B is a group of the formula
$-(CH_2)_n-$,

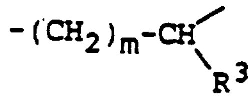 , 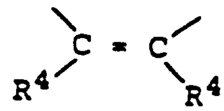

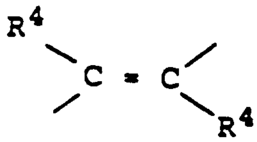 or 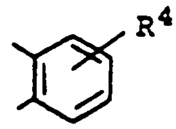

| | |
|---|---|
| n | is from 1 to 20, |
| m | is 0, 1 or 2, |
| $R^3$ | is $C_1$-$C_{20}$-alkyl and |
| $R^4$ | is in each case hydrogen or $C_1$-$C_{20}$-alkyl. |

**2.** Compounds according to claim 1 wherein y is 1 or 2, A is hydrogen, $C_1$-$C_2$-alkyl, phenyl or a group of the formula

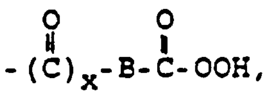

if y is 1, or $C_2$-$C_{20}$-alkylene or phenylene if y is 2,

| | |
|---|---|
| $R^1$ and $R^2$ | can be identical or different and are each hydrogen, $C_1$-$C_{20}$-alkyl, phenyl, chlorophenyl, $C_1$-$C_4$-alkoxyphenyl or $C_1$-$C_4$-alkylphenyl, or else $R^1$ and $R^2$ are together a $C_2$-$C_4$-alkylene radical, |
| x | is 0 or 1, |
| B | is a group of the formula $-(CH_2)_n-$, |

$-(CH_2)_n-$, 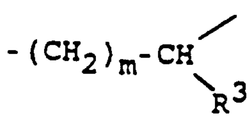 , 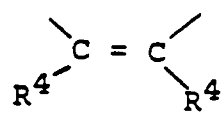

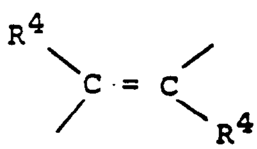 or 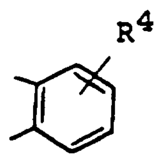

| | |
|---|---|
| n | is from 1 to 20, |
| m | is 0, 1 or 2, |
| $R^3$ | is $C_1$-$C_{20}$-alkyl and |
| $R^4$ | is in each case hydrogen or $C_1$-$C_{20}$-alkyl. |

**3.** Compounds according to claim 1 wherein y is 1, A is hydrogen, phenyl or $C_1$-$C_2$-alkyl, $R^1$ and $R^2$ are each hydrogen, x is zero or 1, B is a group of the formula $-(CH_2)_n-$ and n is from 1 to 20.

**4.** Compounds according to claim 1 wherein y is 1, A is hydrogen, phenyl or $C_1$-$C_2$-alkyl, $R^1$ and $R^2$ are each hydrogen, x is zero, B is a group of the formula $-(CH_2)_n-$, and n is from 1 to 10, preferably from 1 to 6.

**5.** Process for preparing the compounds according to at least one of claims 1 to 4, characterized in that a ureidocarboxylic acid of the formula

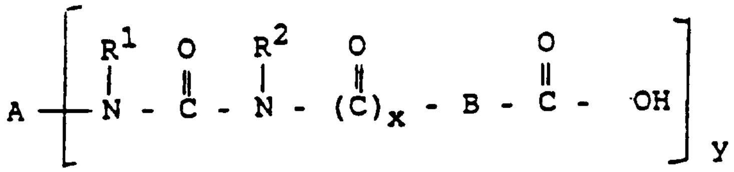

wherein

y is 1 or 2,

A is hydrogen, $C_1$-$C_2$-alkyl, aryl or a group of the formula

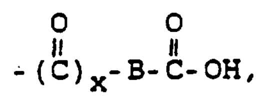

if y is 1, or $C_2$-$C_{20}$-alkylene or arylene if y is 2,

$R^1$ and $R^2$ can be identical or different and are each hydrogen, $C_1$-$C_{20}$-alkyl, aryl, haloaryl, alkoxyaryl or alkylaryl, or else $R^1$ and $R^2$ are together a $C_2$-$C_4$-alkylene radical,

x is 0 or 1,

B is a group of the formula
$-(CH_2)_n-$,

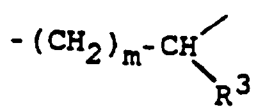
,
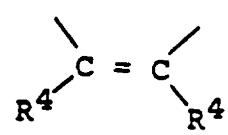

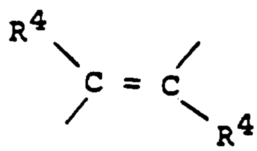
or
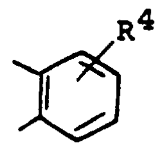

n is from 1 to 20,

m is 0, 1 or 2,

$R^3$ is $C_1$-$C_{20}$-alkyl and

$R^4$ is in each case hydrogen or $C_1$-$C_{20}$-alkyl,

is reacted with an oxidising mixture of hydrogen peroxide with a strong acid, preferably sulfuric acid, methanesulfonic acid or an acidic ion exchange material, hydrogen peroxide being used in a molar ratio of from 10 to 1 : 1, preferably from 4 to 2 : 1, per oxidizable carboxyl group in the ureidocarboxylic acid and the amount of strong acid used being from 2 to 6 times the weight of the ureidocarboxylic acid.

**6.** Process according to claim 5, characterized in that a from 30 to 95 percent strength by weight, preferably from 50 to 85 percent strength by weight, aqueous hydrogen peroxide solution is used.

**7.** Process as set forth in claim 5 or 6, characterized in that the strong acid used is from 50 to 96 percent strength by weight, preferably from 75 to 96 percent strength by weight, sulfuric acid.

**8.** Process as set forth in any one of claims 5 to 7, characterized in that the reaction temperature is between 5 and 30 °C, in particular between 10 and 20 °C.

**9.** Use of the compounds according to claim 1 as bleaching, oxidizing or disinfecting agent.

**Claims for the following Contracting State : ES**

1. Bleaching, oxidizing or disinfecting agent comprising compounds of the formula

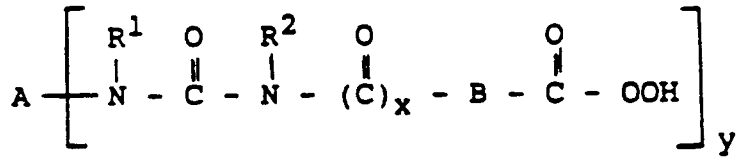

wherein

| | | |
|---|---|---|
| y | is 1 or 2, |
| A | is hydrogen, $C_1$-$C_2$-alkyl, aryl or a group of the formula |

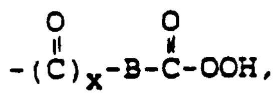

if y is 1, or $C_2$-$C_{20}$-alkylene or arylene if y is 2,

| | |
|---|---|
| $R^1$ and $R^2$ | can be identical or different and are each hydrogen, $C_1$-$C_{20}$-alkyl, aryl, haloaryl, alkoxyaryl or alkylaryl, or else $R^1$ and $R^2$ are together a $C_2$-$C_4$-alkylene radical, |
| x | is 0 or 1, |
| B | is a group of the formula $-(CH_2)_n-$, |

$-(CH_2)_n-$, 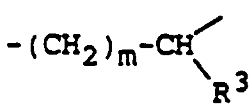 , 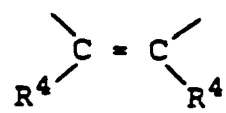

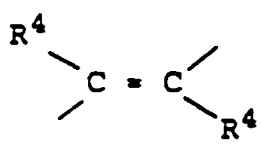 or 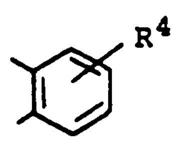

| | |
|---|---|
| n | is from 1 to 20, |
| m | is 0, 1 or 2, |
| $R^3$ | is $C_1$-$C_{20}$-alkyl and |
| $R^4$ | is in each case hydrogen or $C_1$-$C_{20}$-alkyl. |

2. Bleaching, oxidizing or disinfecting agent comprising compounds according to claim 1 wherein y is 1 or 2, A is hydrogen, $C_1$-$C_2$-alkyl, phenyl or a group of the formula

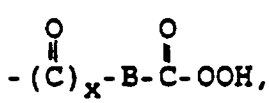

if y is 1, or $C_2$-$C_{20}$-alkylene or phenylene if y is 2,

| | |
|---|---|
| $R^1$ and $R^2$ | can be identical or different and are each hydrogen, $C_1$-$C_{20}$-alkyl, phenyl, chlorophenyl, $C_1$-$C_4$-alkoxyphenyl or $C_1$-$C_4$-alkylphenyl, or else $R^1$ and $R^2$ are together a $C_2$-$C_4$-alkylene radical, |

x is 0 or 1,
B is a group of the formula
$-(CH_2)_n-$,

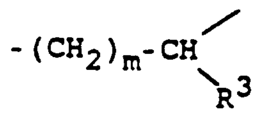 , 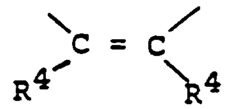

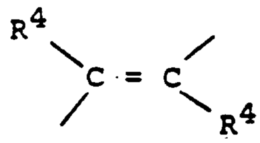 or 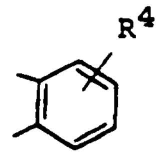 .

n is from 1 to 20,
m is 0, 1 or 2,
$R^3$ is $C_1$-$C_{20}$-alkyl and
$R^4$ is in each case hydrogen or $C_1$-$C_{20}$-alkyl.

3. Bleaching, oxidizing or disinfecting agent comprising compounds according to claim 1 wherein y is 1, A is hydrogen, phenyl or $C_1$-$C_2$-alkyl, $R^1$ and $R^2$ are each hydrogen, x is zero or 1, B is a group of the formula $-(CH_2)_n-$ and n is from 1 to 20.

4. Bleaching, oxidizing or disinfecting agent comprising compounds according to claim 1 wherein y is 1, A is hydrogen, phenyl or $C_1$-$C_2$-alkyl, $R^1$ and $R^2$ are each hydrogen, x is zero, B is a group of the formula $-(CH_2)_n-$ and n is from 1 to 10, preferably from 1 to 6.

5. Process for preparing bleaching, oxidizing or disinfencting agent comprising compounds according to at least one of claims 1 to 4, characterized in that a ureidocarboxylic acid of the formula

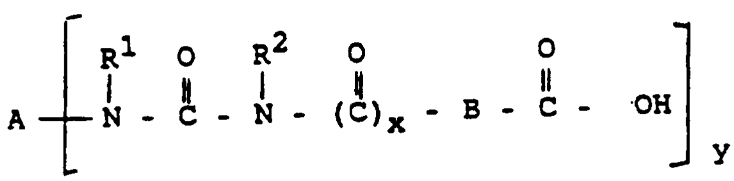

wherein
y is 1 or 2,
A is hydrogen, $C_1$-$C_2$-alkyl, aryl or a group of the formula

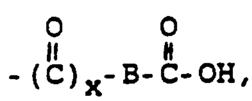

if y is 1, or $C_2$-$C_{20}$-alkylene or arylene if y is 2,
$R^1$ and $R^2$ can be identical or different and are each hydrogen, $C_1$-$C_{20}$-alkyl, aryl, haloaryl, alkoxyaryl or alkylaryl, or else $R^1$ and $R^2$ are together a $C_2$-$C_4$-alkylene radical,
x is 0 or 1,
B is a group of the formula
$-(CH_2)n-$,

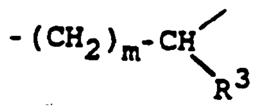 , 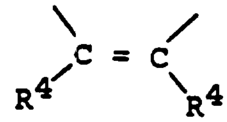

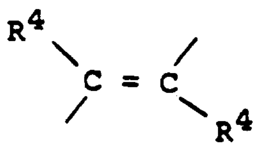 or 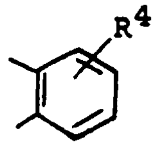

n is from 1 to 20,
m is 0, 1 or 2,
$R^3$ is $C_1$-$C_{20}$-alkyl and
$R^4$ is in each case hydrogen or $C_1$-$C_{20}$-alkyl,

is reacted with an oxidizing mixture of hydrogen peroxide with a strong acid, preferably sulfuric acid, methanesulfonic acid or an acidic ion exchange material, hydrogen peroxide being used in a molar ratio of from 10 to 1 : 1, preferably from 4 to 2 : 1, per oxidizable carboxyl group in the ureidocarboxylic acid and the amount of strong acid used being from 2 to 6 times the weight of the ureidocarboxylic acid.

**6.** Process for preparing bleaching, oxidizing or disinfecting agent comprising compounds according to claim 5, characterized in that a from 30 to 95 percent strength by weight, preferably from 50 to 85 percent strength by weight, aqueous hydrogen peroxide solution is used.

**7.** Process for preparing bleaching, oxidizing or disinfecting agent comprising compounds as set forth in claim 5 or 6, characterized in that the strong acid used is from 50 to 96 percent strength by weight, preferably from 75 to 96 percent strength by weight, sulfuric acid.

**8.** Process for preparing bleaching, oxidizing or disinfecting agent comprising compounds as set forth in any one of claims 5 to 7, characterized in that the reaction temperature is between 5 and 30 °C, in particular between 10 to 20 °C.

**Revendications**

**Revendications pour les Etats contractants suivants : AT, BE, CH, DE, FR, GB, IT, LI, NL**

**1.** Composés de formule

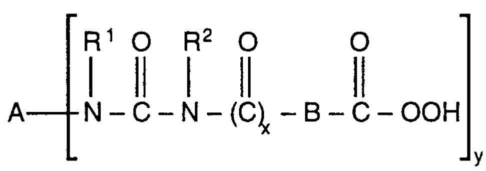

dans laquelle

y signifie les nombres 1 ou 2
A dans le cas y = 1
signifie un hydrogène, un alkyle en $C_1$-$C_2$, un aryle ou un groupe de formule

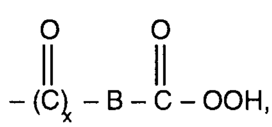

dans le cas y = 2

signifie un alkylène en $C_2$-$C_{20}$, ou un arylène,

$R^1$ et $R^2$ peuvent être identiques ou différents et signifient un hydrogène un alkyle en $C_1$-$C_{20}$, un aryle, un halogénoaryle, un alkoxyaryle, ou un alkylaryle,

ou $R^1$ et $R^2$ peuvent aussi représenter ensemble un reste alkylène en $C_2$-$C_4$,

x signifie les nombres 0 ou 1,

B est un groupe de formule

$-(CH_2)_n-$,

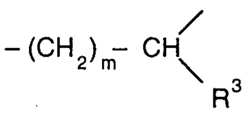
,
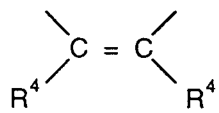

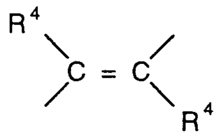
ou
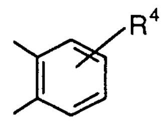

n signifie les nombres allant de 1 à 20,

m signifie les nombres 0, 1 ou 2,

$R^3$ signifie un alkyle en $C_1$-$C_{20}$ et

$R^4$ signifie à chaque fois un hydrogène ou un alkyle en $C_1$-$C_{20}$.

**2.** Composés selon la revendication 1, dans lesquels y signifie les chiffres 1 ou 2

A dans le cas où y = 1, signifie un hydrogène, un alkyle en $C_1$-$C_2$, un phényle ou un groupe de formule

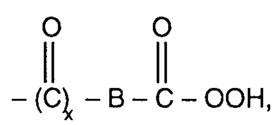

dans le cas où y = 2,

signifie un alkylène en $C_2$-$C_{20}$ ou un phénylène

$R^1$ et $R^2$ peuvent être identiques ou différents et signifient un hydrogène, un alkyle en $C_1$-$C_{20}$, un phényle, un chlorophényle, un (alkoxy en $C_1$-$C_4$)phényle ou un (alkyle en $C_1$-$C_4$)-phényle,

ou $R^1$ et $R^2$ peuvent représenter ensemble un reste alkylène en $C_2$-$C_4$,

x signifie les chiffres 0 ou 1

B signifie un groupe de formule

$-(CH_2)_n-$,

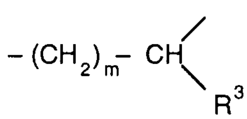
,
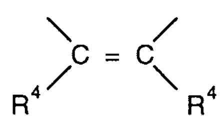

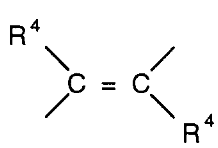
ou
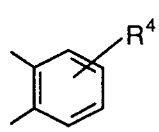

n signifie un nombre allant de 1 à 20,
m signifie les chiffres 0, 1 ou 2,
$R^3$ signifie un alkyle en $C_1$-$C_{20}$ et
$R^4$ signifie à chaque fois un hydrogène ou un alkyle en $C_1$-$C_{20}$.

**3.** Composés selon la revendication 1, dans lesquels y signifie le chiffre 1, A signifie un hydrogène, un phényle ou un alkyle en $C_1$-$C_2$, et $R^1$ et $R^2$ signifient un hydrogène, x les chiffres 0 ou 1, B signifie un groupe de formule $-(CH_2)_n-$ et n signifie les nombres allant de 1 à 20.

**4.** Composés selon la revendication 1, dans lesquels y signifie le chiffre 1, A signifie un hydrogène, un phényle ou un alkyle en $C_1$-$C_2$, et $R^1$ et $R^2$ signifient un hydrogène, x le chiffre 0, B signifie un groupe de formule $-(CH_2)_n-$ et n signifie les nombres allant de 1 à 10, de préférence allant de 1 à 6.

**5.** Procédé de préparation des composés selon au moins une des revendications 1 à 4, caractérisé en ce qu'un acide uréidocarboxylique de formule

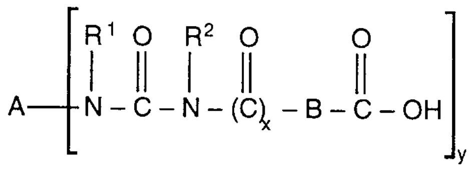

dans laquelle

y signifie les chiffres 1 ou 2
A dans le cas y = 1,
signifie un hydrogène, un alkyle en $C_1$-$C_2$, un aryle, ou un groupe de formule

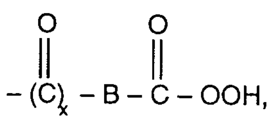

dans le cas où y = 2
signifie un alkylène en $C_2$-$C_{20}$. ou un arylène,
$R^1$ et $R^2$ peuvent être identiques ou différents et signifient un hydrogène, un alkyle en $C_1$-$C_{20}$, un aryle, un halogénoaryle, un alkoxyaryle, ou un alkylaryle,
ou $R^1$ et $R^2$ peuvent aussi représenter ensemble un reste alkylène en $C_2$-$C_4$,
x signifie les chiffres 0 ou 1
B signifie un groupe de formule
$-(CH_2)_n-$,

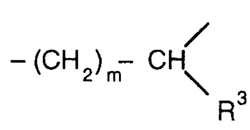 , 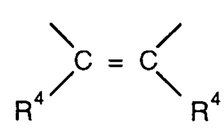

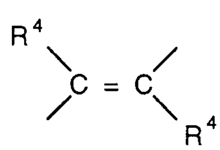 ou 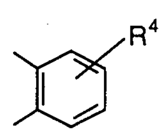

n signifie un nombre allant de 1 à 20,
m signifie les chiffres 0, 1 ou 2,
$R^3$ signifie un alkyle en $C_1$-$C_{20}$ et

$R^4$ signifie à chaque fois un hydrogène ou un alkyle en $C_1$-$C_{20}$.

est mis en réaction avec un mélange d'oxydation à base de peroxyde d'hydrogène avec un acide fort de préférence l'acide sulfurique, l'acide méthane sulfonique ou un échangeur d'ions acides, le peroxyde d'hydrogène étant introduit dans un rapport molaire allant de 10 à 1 par groupe carboxyle oxydable de l'acide uréidocarboxylique, de préférence allant de 4 à 2 et la quantité pondérale de l'acide fort est de 2 à 6 fois la quantité en acide uréidocarboxylique.

**6.** Procédé selon la revendication 5, caractérisé en ce qu'on utilise une solution aqueuse en peroxyde d'hydrogène allant de 30 à 95 % en poids, de préférence de 50 à 85 % en poids.

**7.** Procédé selon la revendication 5 ou 6, caractérisé en ce qu'en tant qu'acide fort, on utilise de l'acide sulfurique en concentration allant de 50 à 96 % en poids, de préférence de 75 à 96 % en poids.

**8.** Procédé selon une des revendications 5 à 7, caractérisé en ce que la température de réaction se situe entre 5 et 30 °C, en particulier entre 10 et 20 °C.

**9.** Utilisation des composés selon la revendication 1 en tant qu'agent de blanchiment, d'oxydation ou de désinfection.

**Revendications pour l'Etat contractant suivant : ES**

**1.** Agents de blanchiment, d'oxydation ou de désinfection contenant des composés de formule

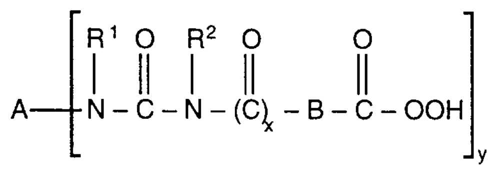

dans laquelle

y signifie les nombres 1 ou 2

A dans le cas y = 1

signifie un hydrogène, un alkyle en $C_1$-$C_2$, un aryle ou un groupe de formule

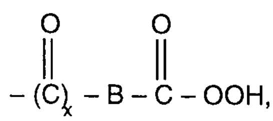

dans le cas y = 2

signifie un alkylène en $C_2$-$C_{20}$, ou un arylène,

$R^1$ et $R^2$ peuvent être identiques ou différents et signifient un hydrogène, un alkyle en $C_1$-$C_{20}$, un aryle, un halogénoaryle un alkoxyaryle ou un alkylaryle,

ou $R^1$ et $R^2$ peuvent aussi représenter ensemble un reste alkylène en $C_2$-$C_4$,

x signifie les nombres 0 ou 1,

B est un groupe de formule

$-(CH_2)_n-$,

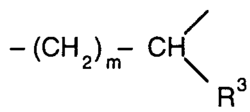 , 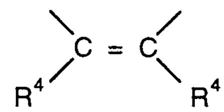

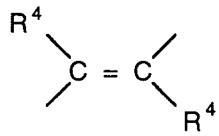 ou 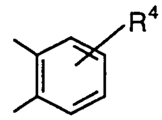

n signifie les nombres allant de 1 à 20,
m signifie les nombres 0, 1 ou 2,
$R^3$ signifie un alkyle en $C_1$-$C_{20}$ et
$R^4$ signifie à chaque fois un hydrogène ou un alkyle en $C_1$-$C_{20}$.

**2.** Agents de blanchiment, d'oxydation ou de désinfection contenant des composés selon la revendication 1, dans lesquels y signifie les chiffres 1 ou 2
A dans le cas où y = 1, signifie un hydrogène, un alkyle en $C_1$-$C_2$, un phényle ou un groupe de formule

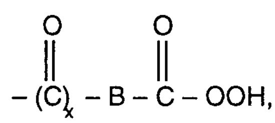

dans le cas où y = 2,
signifie un alkylène en $C_2$-$C_{20}$ ou un phénylène
$R^1$ et $R^2$ peuvent être identiques ou différents et signifient un hydrogène, un alkyle en $C_1$-$C_{20}$, un phényle, un chlorophényle, un (alkoxy en $C_1$-$C_4$)phényle ou un (alkyle en $C_1$-$C_4$)-phényle,
ou $R^1$ et $R^2$ peuvent représenter ensemble un reste alkylène en $C_2$-$C_4$,
x signifie les chiffres 0 ou 1
B signifie un groupe de formule
$-(CH_2)_n-$,

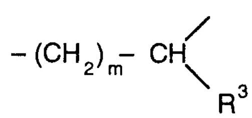 , 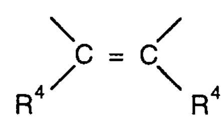

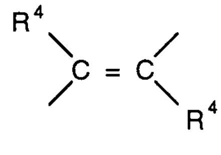 ou 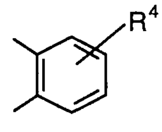

n signifie un nombre allant de 1 à 20,
m signifie les chiffres 0, 1 ou 2,
$R^3$ signifie un alkyle en $C_1$-$C_{20}$ et
$R^4$ signifie à chaque fois un hydrogène ou un alkyle en $C_1$-$C_{20}$.

**3.** Agents de blanchiment, d'oxydation ou de désinfection contenant des composés selon la revendication 1, dans lesquels y signifie le chiffre 1, A signifie un hydrogène, un phényle ou un alkyle en $C_1$-$C_2$, et

$R^1$ et $R^2$ signifient un hydrogène, x les chiffres 0 ou 1, B signifie un groupe de formule $-(CH_2)_n-$et n signifie les nombres allant de 1 à 20.

**4.** Agents de blanchiment, d'oxydation ou de désinfection contenant des composés selon la revendication 1, dans lesquels y signifie le chiffre 1, A signifie un hydrogène, un phényle ou un alkyle en $C_1$-$C_2$, et $R^1$ et $R^2$ signifient un hydrogène, x le chiffre 0, B signifie un groupe de formule $-(CH_2)_n-$ et n signifie les nombres allant de 1 à 10, de préférence allant de 1 à 6.

**5.** Procédé de préparation d'agents de blanchiment, d'oxydation ou de désinfection contenant des composés selon au moins une des revendications 1 à 4, caractérisé en ce qu'un acide uréidocarboxylique de formule

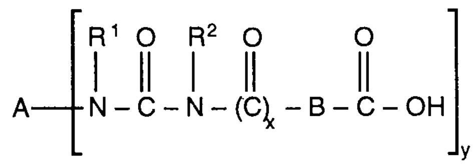

dans laquelle

y signifie les chiffres 1 ou 2

A dans le cas y = 1,
signifie un hydrogène, un alkyle en $C_1$-$C_2$, un aryle, ou un groupe de formule

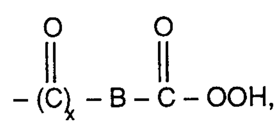

dans le cas où y = 2
signifie un alkylène en $C_2$-$C_{20}$. ou un arylène,

$R^1$ et $R^2$ peuvent être identiques ou différents et signifient un hydrogène, un alkyle en $C_1$-$C_{20}$, un aryle, un halogénoaryle, un alkoxyaryle, ou un alkylaryle,
ou $R^1$ et $R^2$ peuvent aussi représenter ensemble un reste alkylène en $C_2$-$C_4$)

x signifie les chiffres 0 ou 1

B signifie un groupe de formule
$-(CH_2)_n-$,

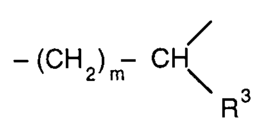
,
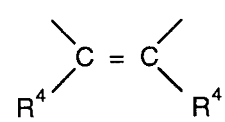

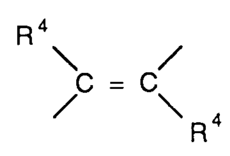
ou
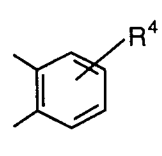

n signifie un nombre allant de 1 à 20,

m signifie les chiffres 0, 1 ou 2,

$R^3$ signifie un alkyle en $C_1$-$C_{20}$ et

$R^4$ signifie à chaque fois un hydrogène ou un alkyle en $C_1$-$C_{20}$.

est mis en réaction avec un mélange d'oxydation à base de peroxyde d'hydrogène avec un acide fort de préférence l'acide sulfurique, l'acide méthane sulfonique ou un échangeur d'ions acides, le peroxyde d'hydrogène étant introduit dans un rapport molaire allant de 10 à 1 par groupe carboxyle

oxydable de l'acide uréidocarboxylique, de préférence allant de 4 à 2 et la quantité pondérale de l'acide fort est de 2 à 6 fois la quantité en acide uréidocarboxylique.

**6.** Procédé de préparation d'agents de blanchiment, d'oxydation ou de désinfection contenant des composés selon la revendication 5, caractérisé en ce qu'une solution aqueuse de peroxyde d'hydrogène allant de 30 à 95 % en poids, de préférence de 50 à 85 % en poids est utilisée.

**7.** Procédé de préparation d'agents de blanchiment, d'oxydation ou de désinfection contenant des composés selon la revendication 5 ou 6, caractérisé en ce qu'en tant qu'acide fort, on utilise de l'acide sulfurique en concentration allant de 50 à 96 % en poids, de préférence de 75 à 96 % en poids.

**8.** Procédé de préparation d'agents de blanchiment, d'oxydation ou de désinfection selon une des revendications 5 à 7, caractérisé en ce que la température de réaction se situe entre 5 et 30 ° C, en particulier entre 10 et 20 ° C.